# EUROPÄISCHE PATENTSCHRIFT

(11) **EP 2 956 177 B1**
(45) Veröffentlichungstag und Bekanntmachung des Hinweises auf die Patenterteilung: **09.10.2019**
(21) Anmeldenummer: 14701289.2
(22) Anmeldetag: 22.01.2014
(51) Int. Cl.: A61L 2/26, E06B 7/23

(54) **LIPPENDICHTUNG ZUR VERWENDUNG BEI EINEM SICHTFENSTER**
SEALING LIP FOR USE WITH A VIEWING WINDOW
FENÊTRE D'INSPECTION MÉNAGÉE DANS UNE ENCEINTE, JOINT À LÈVRE CONÇU POUR CETTE FENÊTRE D'INSPECTION, ET ENCEINTE

(30) Priorität: 12.02.2013 DE 102013101384
(43) Veröffentlichungstag der Anmeldung: 23.12.2015
(73) Patentinhaber: KHS GmbH, 44143 Dortmund (DE)
(72) Erfinder: VORWERK, Jürgen, 56290 Mörsdorf (DE); NIEHR, Thomas, 55583 Bad Münster am Stein Ebernburg (DE); SCHEUFEN, Marcel, 55543 Bad Kreuznach (DE)
(86) Internationale Anmeldenummer: PCT/EP2014/000162
(87) Internationale Veröffentlichungsnummer: WO 2014/124727

(56) Entgegenhaltungen:
- EP-A2- 1 614 850
- WO-A1-00/43625
- WO-A1-00/43625
- WO-A1-2009/003924
- AT-B- 343 335
- AT-B- 343 335
- CH-A5- 581 775
- CH-A5- 581 775
- DE-A1- 1 683 650
- DE-A1- 2 340 731
- DE-A1- 2 340 731
- DE-A1- 3 315 091
- DE-A1- 3 315 091
- DE-A1- 3 524 124
- DE-B- 1 087 792
- DE-B- 1 087 792
- DE-U1- 9 015 034

## Beschreibung

Die Erfindung bezieht sich auf ein Sichtfenster gemäß Oberbegriff Patentanspruch 1 sowie auf eine Einhausung gemäß Oberbegriff Patentanspruch 9. Insbesondere in der Getränkeindustrie ergeben sich zunehmend erhöhte Anforderungen an die Hygiene bei der Behandlung von Packmitteln, insbesondere beim Füllen und Verschließen von Packmitteln unter sterilen und/oder aseptischen Bedingungen. Entsprechende Behandlungsmaschinen oder -vorrichtungen erfordern daher häufig, dass bei der Behandlung die Packmittel zumindest an ihrem die jeweilige Packmittelöffnung aufweisenden Bereich in einem eine Aseptikzone bildenden Rein- oder Sterilraum einer Einhausung, die diesen Rein- oder Sterilraum von einer nicht sterilen Umgebung trennt. Um für Wartungs- und Reparaturarbeiten einen Zugang zu ermöglichen und die Behandlungsprozesse der Packmittel, beispielsweise die Füll- und Verschließprozesse innerhalb des Rein- oder Sterilraumes von Außen ohne Öffnen der Einhausung optisch überwachen zu können, ist es üblich, in den die Einhausung bildenden Trennwänden Sichtfenster vorzusehen. Dabei ist in zunehmendem Maße entscheidend, dass der wesentliche Teil der Trennwände transparent ausgeführt wird. Diese bestehen grundsätzlich aus Einbaurahmen und Scheiben, die abnehmbar, aber unter Verwendung von dauerelastischen Scheibendichtungen dicht mit den Einbaurahmen verbunden sind. Die Scheibendichtungen sind dabei durch Klebeverbindungen oder unter Verwendung gesonderte Klemmprofile mit dem jeweiligen Einbaurahmen verbunden. An die verwendeten Scheibendichtungen bzw. an die diese Dichtungen aufweisenden Dichtungsanordnungen zwischen dem jeweiligen Sichtfenster und dem Einbaurahmen sind hohe Anforderungen zu stellen, da sie entscheidend u.a. die sterilen und/oder aseptischen Bedingungen innerhalb des Aseptikzone bildenen Rein- oder Sterilraums bestimmen.

Die bisher üblichen Scheibendichtungen und Dichtungsanordnungen haben den Nachteilig hierbei ist u.a., dass sich im Bereich der Scheibendichtungen unkontrollierte Ecken, Spalten und/oder Hinterschneidungen und/oder Taschen, die regelmäßig Ausgangspunkt für Verkeimungen und/oder Verschmutzungen bilden, insbesondere auch an der dem Rein- oder Sterilraumraum zugewandten Seite der jeweiligen Scheibenabdichtung nicht vermeiden lassen. Das Phänomen der Taschenbildung wird dabei durch den Chemikalieneinwirkung und hohe Temperaturen sowie intensive Luftströmungen begünstigt. Insbesondere mit zunehmender Einsatzzeit erhöht sich dieses Risiko, weil die dichtungseigene Klemmwirkung nachlässt bzw. Klebeverbindungen angegriffen werden. Klebeverbindungen mit Klemmprofilen zum Halten der Scheibenabdichtungen bedeuten außerdem eine umständliche und zeitaufwendige Montage. Gleiches gilt grundsätzlich auch für Klebeverbindungen, die darüber hinaus beim Austausch von defekten Sichtfenstern oder defekter Dichtungen unerwünscht sind.

Bekannt sind eine Lippendichtung mit den Merkmalen des Oberbegriffs des Patentanspruchs 1 sowie ein Sichtfenster mit den Merkmalen des Oberbegriffs des Patentanspruchs 3 (DE 102 35 374 A1). Die Befestigung der Lippendichtung an dem eine Öffnung einer Einhausung umgebenden Öffnungsrand erfolgt im bekannten Fall dadurch, dass sie an ihrem für die Befestigung dienenden Lippendichtungsabschnitt als U-Profil ausgeführt ist und mit diesem Lippendichtungsabschnitt einen Öffnungsrand der Öffnung des Sichtfensters reiterartig übergreift. Eine zuverlässige und insbesondere auch dauerhaft spaltfreie Befestigung der Lippendichtung an der Einhausung ist hiermit nicht erreichbar.

Bekannt sind weiterhin aufblasbare Dichtungsprofile (GB 2 273 960 A) zum Abdichten des Übergangs zwischen einem Türflügel und einem diesen umgebenden Rahmen. Die Dichtungsprofile sind dabei teilweise auch mit einem sichelartigen Dichtungsabschnitt versehen, der sich beim Aufblasen des Dichtungsprofils gegen den Türflügel anlegt. Für eine umlaufende Dichtung eines Öffnungsverschlusses und Sichtfensters einer einen Rein- oder Sterilraum bildenden Einhausung sind diese bekannten Dichtungsprofile weder bestimmt, noch geeignet.

Aufgabe der Erfindung ist es, eine Dichtung aufzuzeigen, mit welcher die vorgenannten Nachteile vermeiden werden und die u.a. eine Scheibenabdichtung bei Einhausungen in der Weise ermöglicht, dass unerwünschte Ecken, Spalten und/oder Hinterschneidungen insbesondere auch an der dem Rein- oder Sterilraum der Einhausung zugeordneten Seite wirksam vermieden sind, insbesondere auch während der Dauer des geschlossenen Sichtfensters.

Zur Lösung dieser Aufgabe wird ein Sichtfenster nach Anspruch 1 vorgeschlagen.

Die erfindungsgemäße Lippendichtung bzw. eine diese Dichtung aufweisende Dichtungsanordnung erfüllen die hohen Anforderungen auch bei Einhausungen für Steril- oder Reinräume in optimaler Weise. Durch die spezielle Formgebung der Lippendichtung ist auch die Möglichkeit einer fehlerhaften Dichtungsmontage vermieden werden bzw. minimiert.

Für ihre Befestigung ist die vorzugsweise als Vollprofil ausgeführte Lippendichtung mit ihrem ersten Lippendichtungsabschnitt elastisch verformt zwischen zwei parallelen oder im Wesentlich parallelen Einspannflächen eingespannt, so dass auch im Bereich dieser Lippendichtungsbefestigung unkontrollierte Ecken, Spalten und/oder Hinterschneidungen und/oder Taschen vermieden sind, die Ausgangspunkt für Verkeimungen und/oder Verschmutzungen bilden könnten.

"Als Vollprofil ausgeführte Lippendichtung" bedeutet im Sinne der Erfindung, dass die Lippendichtung keine Ausnehmung bzw. Nut für eine Befestigung durch Aufschieben auf einen Öffnungsrand (diesen reiterartig übergreifend) aufweist, wie dies bei bekannten Dichtungen u.a. der Fall ist.

"Umlaufend" bedeutet im Sinne der Erfindung, dass sich die jeweilige Lippendichtung oder ein an dieser Dichtung ausgebildeter Wulst um den gesamten Umfang der Einbaurahmenöffnung erstreckt und dabei bevorzugt durchgehend, d.h. ohne Unterbrechung ausgebildet ist.

"Packmittel" sind im Sinne der Erfindung Verpackungen oder Behältnisse, die insbesondere im Lebensmittelbereich und dabei speziell auch im Getränkebereich üblicherweise verwendet werden, und zwar u.a. Behälter, wie z. B. Flaschen, Dosen, auch Weichverpackungen, beispielsweise solche hergestellt aus Karton und/oder Kunststofffolie und/oder Metallfolie usw.

"Behandlungsmaschinen" oder "Behandlungsvorrichtungen" im Sinne der Erfindung sind Maschinen oder Vorrichtungen zum Behandeln und/oder Verarbeiten von Packmitteln, insbesondere Blasformmaschinen zum Blasformen von Behältern, Reinigungsmaschinen, Rinser, Füllmaschinen und Verschließmaschinen.

"Rein- oder Sterilraum" ist im Sinne der vorliegenden Erfindung bevorzugt ein eine Aseptikzone bildender Raum, in dem für eine Behandlung unter sterilen und/oder aseptischen Bedingungen die Packmittel zumindest mit ihrem die jeweilige Packmittelöffnung aufweisenden Bereich aufgenommen sind.

Der Ausdruck "im Wesentlichen" bzw. "etwa" bedeutet im Sinne der Erfindung Abweichungen vom jeweils exakten Wert um +/- 10%, bevorzugt um +/- 5% und/oder Abweichungen in Form von für die Funktion unbedeutenden Änderungen.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an einem Ausführungsbeispiel näher erläutert. Es zeigen:

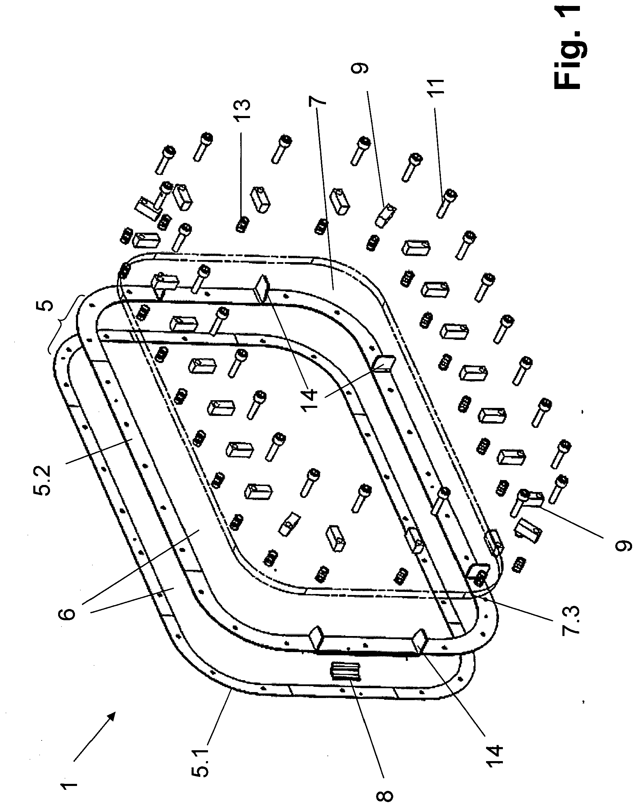

- Fig. 1: in einer Explosionsdarstellung die Elemente eines Sichtfensters einer Einhausung für die einer Anlage zur sterilen und/oder aseptischen Behandlung von Behältern;
- Fig. 2 u. 3: das Sichtfenster der Figur 1 im zusammengebauten Zustand in Draufsicht sowie in Seitenansicht;
- Fig. 4: einen Schnitt entsprechend der Linie I - I der Figur 2
- Fig. 5: in vergrößerter Teildarstellung und im Schnitt den Rahmen des Sichtfensters der Figuren 1 - 4 zusammen mit der die Fenster- oder Rahmenöffnung verschließenden Scheibe;
- Fig. 6: in Detaildarstellung eine Lippendichtung zur Verwendung bei dem Sichtfenster der Figuren 1 - 5;
- Fig. 7: in vergrößerter Darstellung den Übergang des im Einbaurahmen unter elastischer Verformung eingespannten Lippendichtungsabschnitts und des nicht eingespannten freien Lippendichtungsabschnitts.

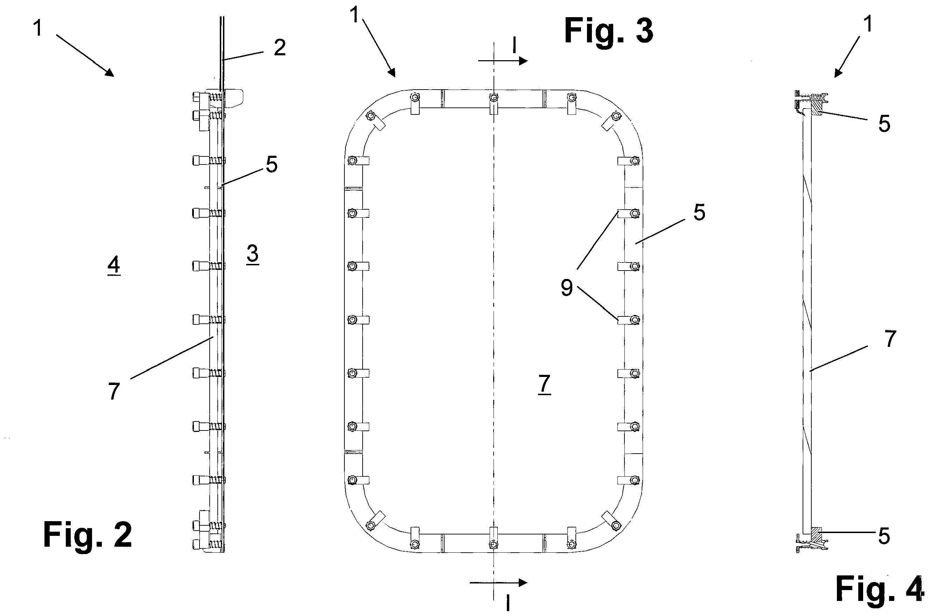

Das in den Figuren allgemein mit 1 bezeichnete Sichtfenster ist Bestandteil einer Einhausung von Behandlungsmaschinen oder -vorrichtungen oder -aggregaten zur sterilen und/oder aseptischen Behandlung von Packmitteln, z.B. zum sterilen und/oder aseptischen Füllen und Verschließen der Packmittel mit einem Füllgut. Die Einhausung, von der in der Figur 3 lediglich eine vertikale Trennwand 2 dargestellt ist, trennt einen eine Aseptikzone bildenden Rein- oder Sterilraum 3, in dem die Packmittel bei ihrer Behandlung zumindest an ihrem die jeweilige Packmittelöffnung aufweisenden Bereich aufgenommen sind, von einer nicht sterilen Umgebung 4.

Das Sichtfenster 1 umfasst im Wesentlichen einen Fenster- oder Einbaurahmen 5, der in geeigneter Weise abgedichtet in einer in der Trennwand 2 vorgesehenen und an die Form des Einbaurahmens 5 angepassten Trennwandöffnung befestigt ist, eine Scheibe 7, die die Einbaurahmenöffnung 6 des Einbaurahmens 5 verschließt und aus einem transparenten, glasklarem Material, beispielsweise Glas oder Kunststoff, z.B. aus Polycarbonat besteht, eine Lippendichtung 8, die als Scheibendichtung den Übergang zwischen dem Einbaurahmen 5 und der Scheibe 7 abdichtet, sowie eine Vielzahl von Riegeln 9, mit denen die Scheibe 7 am Einbaurahmen 5 abnehmbar und gegen die Lippendichtung 8 angedrückt gehalten ist, und zwar ohne dass Befestigungsöffnungen in der Scheibe 7 und/oder Funktionselement an der Scheibe 7 selbst für die Scheibenbefestigung erforderlich sind. Die Lippendichtung 8 ist umlaufend ausgebildet. Die Scheibe 7 bildet eine dem Rein- oder Sterilraum 3 zugewandte oder zugeordnete Scheibeninnenseite 7.1 und eine der Umgebung 4 zugewandte oder zugeordnete Scheibenaußenseite 7.2.

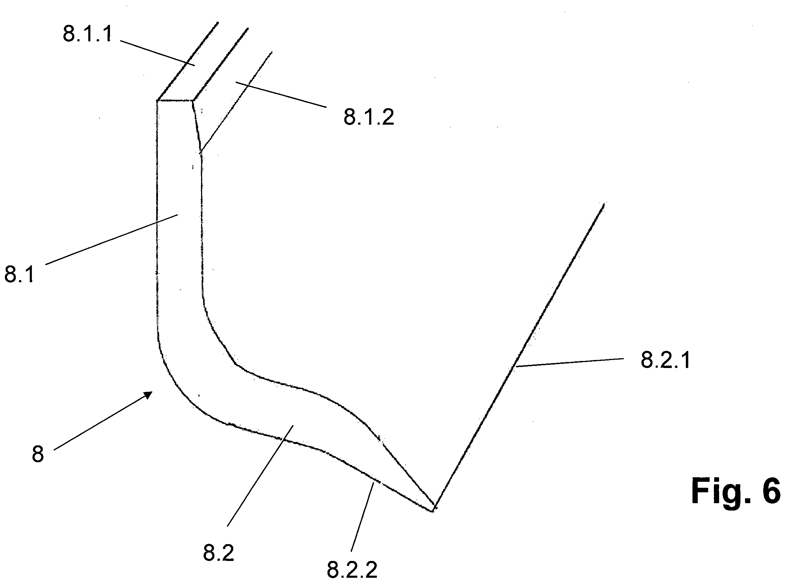

Die Lippendichtung 8, die aus einem gummi- oder dauerelastischen Material, vorzugsweise aus einem gummi- oder dauerelastischen Material mit Lebensmittelzulassung, z.B. aus einem entsprechenden Kunststoff, beispielsweise aus Silikon besteht, ist in der Figur 6 im Detail dargestellt. Sie besitzt im Ursprungszustand, d.h. im nicht verformten Zustand einen im Wesentlichen sichelartigen Profilquerschnitt mit einem ersten, leistenartig ausgebildeten Lippendichtungsabschnitt 8.1 und mit einem zweiten Lippendichtungsabschnitt 8.2, der in einem an den ersten Lippendichtungsabschnitt 8.1 anschließenden ersten Teilabschnitt 8.1.1 des Profilquerschnitts um wenigstens eine in Längsrichtung der Lippendichtung orientierte Achse gekrümmt ist und den dem ersten, Lippendichtungsabschnitt 8.1 entfernten Dichtungslippenrand 8.2.3 bildet. Die Dicke des zweiten Lippendichtungsabschnitts 8.2 nimmt dabei zu dem Dichtungslippenrand 8.2.3 hin ab.

Mehr in Detail ist eine erste Seite des Profilquerschnitts der Lippendichtung 8 an dem ersten Teilabschnitt 8.2.1 um wenigstens eine in Längsrichtung der Lippendichtung 8 orientierte Achse konvex und in einem an den ersten Teilabschnitt 8.2.1 anschließenden, den Dichtungslippenrand 8.2.3 aufweisenden zweiten Teilabschnitt 8.2.2 des zweiten Lippendichtungsabschnitts 8.2 um wenigstens eine in Längsrichtung der Lippendichtung 8 orientierte Achse konkav gekrümmt ist. Die andere Seite des Profilquerschnitts der Lippendichtung 8 ist an dem ersten Teilabschnitt 8.2.1 um wenigstens eine in Längsrichtung der Lippendichtung orientierte Achse konkav und in einem an den ersten Teilabschnitt 8.2.1 anschließenden, den Dichtungslippenrand (8.2.3) aufweisenden zweiten Teilabschnitt 8.2.2 um wenigstens eine in Längsrichtung der Lippendichtung 8 orientierte Achse konvex gekrümmt. Der leistenartige Lippendichtungsabschnitt 8.1 besitzt eine gleichbleibende Dicke und ist lediglich an seinem dem Lippendichtungsabschnitt 8.2 entfernt liegenden Randbereich 8.1.1 an der dem Lippendichtungsabschnitt 8.2 zugewandten Innenseite bei 8.1.2 abgeschrägt, sodass dort die Dicke des Lippendichtungsabschnitts 8.1 zum Rand 8.1.1 hin abnimmt.

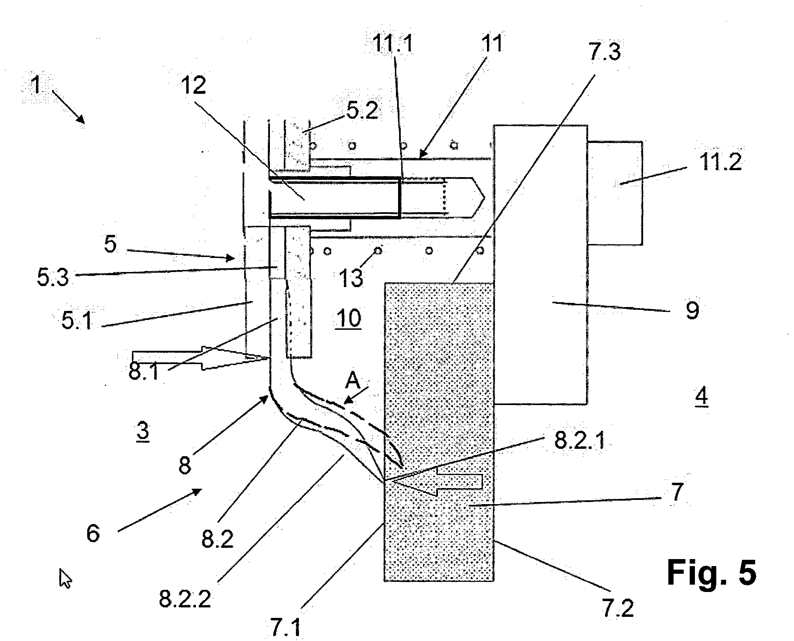

Wie insbesondere die Figur 5 zeigt, ist die am Einbaurahmen 5 gehaltene Scheibe 7 gegenüber diesem Einbaurahmen bzw. dessen Ebene in Richtung zur Umgebung 4 hin versetzt. Außerdem weist die Scheibe 7 bei der dargestellten Ausführungsform eine Größe auf, die größer ist als die Einbaurahmenöffnung 6, sodass die Scheibe 7 mit ihrem Schreibenrand 7.3 in Blickrichtung senkrecht zur Ebene Einbaurahmenöffnung 6 den Einbaurahmen 5 im Bereich des Randes der Einbaurahmenöffnung 6 überlappt, zwischen dem Einbaurahmen 5 und der Scheibe 7 aber ein Spalt 10 gebildet ist. Die Scheibe 7 ist somit mit ihrer Scheibeninnenseite 7.1 der Außenseite des Einbaurahmens 5 zugewandt, aber von dieser Ebene beabstandet.

Der Einbaurahmen 5 besteht bei der dargestellten Ausführungsform aus zwei rahmenartigen, die Einbaurahmenöffnung 6 umschließenden Rahmenelementen oder -teilen 5.1 und 5.2 aus einem metallischen Werkstoff, beispielsweise aus korrosionsbeständigem Stahl, oder aus Kunststoff. Zwischen den beiden Rahmenteilen 5.1 und 5.2, die in Richtung einer senkrecht zur Ebene der Einbaurahmenöffnung 6 orientierten Achse hintereinander bzw. aufeinander folgend vorgesehen sind, ist die Lippendichtung 8 mit dem Lippendichtungsabschnitt 8.1 eingespannt, und zwar vorzugsweise ohne Verkleben und unter elastischer Verformung des Materials der Lippendichtung 8, derart, dass der eingespannte Lippendichtungsabschnitt 8.1 eine Dicke aufweist, die nur noch etwa 80%-90% der ursprünglichen Dicke entspricht, die der Lippendichtungsabschnitt 8.1 im nicht eingespannten Zustand besitzt. Der Abstand zwischen den beiden Rahmenteilen 5.1 und 5.2, d.h. die Breite des Einbauraums oder Einbauspaltes 5.3 für den Lippendichtungsabschnitt 8.1 beträgt dabei beispielsweise 1,8 - 2,2 mm, vorzugsweise 2 mm. Der aus dem Einbauspalt 5.3 an dem die Einbaurahmenöffnung 6 umschließenden Innenrand des Einbaurahmens 5 vorstehende und in die die Einbaurahmenöffnung 6 hinein reichende Lippendichtungslabschnitt 8.2 ist gekrümmt, und zwar bei montierter Scheibe 7 an seiner dem Rein- oder Sterilraum 3 zugewandten Seite oder Fläche ausgehend von dem Innenrand des Einbaurahmens 5 zunächst konvex und anschließend konkav. Der Lippendichtungslabschnitt 8.2 liegt mit seinem freien, sich verjüngenden Ende bzw. mit einem dortigen umlaufenden Dichtungsrand 8.2.3 angepresst gegen die Scheibeninnenseite 7.1 an.

Der Lippendichtungsabschnitt 8.2, der den Spalt 10 abdichtet, ist im Ursprungszustand der Lippendichtung 8, d.h. bei noch nicht eingebauter oder nicht gegen die Scheibe 7 angepresster Lippendichtung 8 stärker gekrümmt, sodass er beim Einbau der Scheibe 7 in Bezug auf seine Krümmung elastisch verformt wird (Pfeil A) und hierdurch die erforderliche Anpresskraft des Dichtungsrandes 8.2.3 gegen die Scheibeninnenseite 7.1 erreicht wird. Von besonderer Bedeutung ist hierbei auch, dass der Lippendichtungsabschnitt 8.2 ohne Ausbildung von Ecken, Spalten und/oder Hinterschneidungen im Rein- oder Sterilraum 3 gegen die Scheibeninnenseite 7.1 anliegt. Dies wird insbesondere durch die spezielle Krümmung des Lippendichtungsabschnitts 8.2 sowie dadurch erreicht wird, dass sich die Dicke der Lippendichtung 8 zum Dichtungsrand 8.2.3 verjüngt, die dem Rein- oder Sterilraum 3 zugewandte Innenfläche 8.2.2 des Lippendichtungsabschnittes 8.2 am Dichtungsrand 8.2.3 stufenlos oder im Wesentlichen stufenlos in die Ebene der Scheibeninnenseite 7.1 übergeht und die Fläche 8.2.2 insbesondere auch am Dichtungsrand 8.2.3 einen Winkel größer als 90° mit der Scheibeninnenseite 7.1 einschließt, der sich zum Rein- oder Sterilraum 3 hin öffnet.

Das Verspannen des Lippendichtungsabschnitts 8.1 zwischen den beiden Rahmenteilen 5.1 und 5.2 erfolgt mit einer Vielzahl von Bolzen 11 mit einem an einem Bolzenende zugänglichen Innengewinde 11.1 und mit einem Bolzenkopf 11.2 am anderen Bolzenende sowie mit Spannschrauben oder Bolzen 12, die am Rahmenteil 5.1 angeschweißt und durch Öffnungen im Rahmenteil 5.2 hindurchgeführt sind und jeweils in ein Innengewinde 11.1 eines Bolzens 11 eingreifen. An den mit ihren Achsen senkrecht zur Ebene des Einbaurahmens 5 orientierten Bolzen 11 sind die Riegel 9 schwenkbar vorgesehen und gegen unerwünschtes Verdrehen durch jeweils eine den zugehörigen Bolzen 11 umschließende Druckfeder 13 gesichert, die sich gegen den Einbaurahmen 5 und den zugehörigen Riegel 9 abstützt. Mit 14 sind Stützelemente in Form von Laschen bezeichnet, die an dem Rahmenteil 5.2 vorgesehen sind und gegen die die Scheibe 7 mit ihrem Scheibenrand 7.3 anliegt.

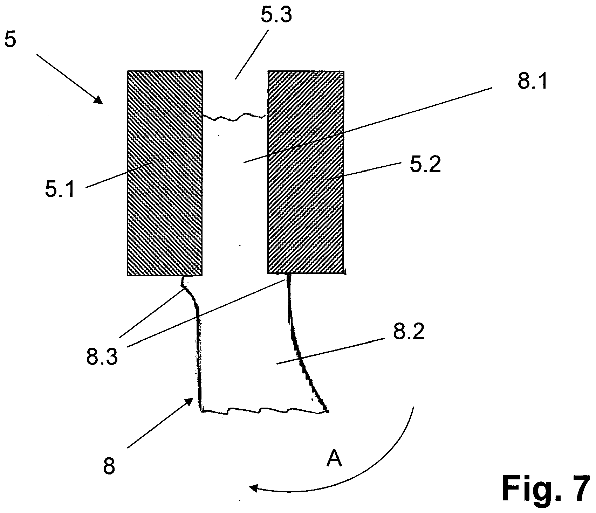

Durch die spezielle Form der Lippendichtung 8 und deren Einbau wird erreicht, dass diese Dichtung zusätzlich zu der mechanischen Verpressung zwischen den Rahmenelementen 5.1 und 5.2 bzw. im Einbauspalt 5.3 bei geschlossenem Fenster gegen die entscheidenden Flächen optimal angepresst wird, d.h. mit dem Dichtungsrand 8.2.3 gegen die Scheibeninnenseite 7.1 und mit dem Lippendichtungsabschnitt 8.1 zusätzlich gegen das Rahmenteil 5.1 an der Innenseite der Einhausung, wie dies in der Figur 7 durch die Pfeile B angedeutet ist.

Durch die besondere Formgebung der Lippendichtung 8 wird auch erreicht, dass deren Lippendichtungsabschnitt 8.2 an einem unteren, horizontalen Bereich der umlaufenden Dichtung an der Innenseite der Einhausung eine schräg nach unten geneigte Fläche bildet, an der in optimaler Weise Flüssigkeit abfließen kann, sodass auch hierdurch verschmutzte und die Gefahr einer Verkeimung bedingende Bereiche vermieden sind.

Der beschriebene Einbau der Lippendichtung 8 hat weiterhin auch den Vorteil, dass die Art und Weise der Befestigung der Lippendichtung selbst bei dünnwandigen Bauteilen oder Komponenten möglich ist, ohne dass diese von der Dichtung umschlossen werden müssen, was in der Regel zu eine unerwünschten Bildung von Taschen führen würde. Die umlaufend ausgeführte Lippendichtung liegt mit ihrem Umfang und auch mit einem wesentlichen Teil ihres Lippendichtungsabschnitts 8.1 außerhalb des Rein- oder Sterilraumes 13, und zwar zwischen den beiden Rahmenteilen 5.1 und 5.2 verpresst, während der Dichtungslippenabschnitt 8.2, der den wirksamen Teil der Lippendichtung 8 bildet, frei stehend und ohne oder im Wesentlichen ohne Einwirkung auf den Einbaurahmen oder die Trennwand 2 ist, sodass auch die Scheibe 7 ohne einen negativen Einfluss, insbesondere auch auf die Abdichtung zwischen der Lippendichtung 8 und dem Einbaurahmen 5 bzw. der Einhausung 2 montiert werden kann.

### Weitere Vorteile des Sichtfensters 1 sind u.a.:

Durch die spezielle Form und die spezielle Art des Einbaus der Lippendichtung 8 werden Ecken, Spalten und/oder Hinterschneidungen im Bereich des Übergangs zwischen dem Einbaurahmen 5 und der Scheibe 7 im Rein- oder Sterilraum 3 zuverlässig vermieden, insbesondere wird durch die Ausbildung der Lippendichtung 8 mit den beiden Lippendichtungsabschnitten 8.1 und 8.2 und durch die Verformung des Lippendichtungsabschnitts 8.2 beim Montieren der Scheibe 7 der den Übergang zwischen der Lippendichtung 8 und dem Einbaurahmen 5 zusätzlich abdichtende Anpressung erzeugt sowie zugleich auch erreicht, dass die Lippendichtung 8 an ihrem Dichtungsrand 8.2.3 ohne Ausbildung von Ecken, Spalten und/oder Hinterschneidungen gegen die Scheibeninnenseite 7.1 anliegt.

Da die Lippendichtung 8 durch Einspannen des Lippendichtungsabschnitts 8.1 zwischen den Rahmenteilen 5.1 und 5.2 gehalten ist, und zwar unter beachtlicher elastischer Verformung dieses Abschnitts, ist auch dort ein dichter Übergang zwischen de Lippendichtung 8 und dem Einbaurahmen 5 gewährleistet.

Durch die stärkere Gesamtkrümmung, die der Dichtungslippenabschnitt 8.2 im ursprünglichen Zustand aufweist und die beim Einbau des Fensters 7 elastisch geändert wird, ergibt sich eine ausrechend hohe Anpresskraft des Dichtungsrandes 8.2.3 gegen die Scheibeninnenseite 7.1.

Die Scheibe 7 ist an ihren Scheibeninnen- und -außenseiten 7.1 und 7.2 vorzugsweise völlig glatt ausgebildet und weist keine eigenen Dichtungselemente oder andere Funktionselemente, wie z.B. Leisten usw. auf, die unerwünschte Ecken, Spalten und/oder Hinterschneidungen bilden könnten.

Die Spannung, mit der der Dichtungslippenabschnitt 8.1 zwischen den Rahmenteilen 5.1 und 5.2 eingespannt ist, wird ausschließlich durch die Bolzen 11 und die Spannschrauben 12 aufgebracht, sodass sich für den Einbau der Lippendichtung 8 reproduzierbare Bedingungen erzielen lassen. Dies gilt auch hinsichtlich der Größe der elastischen Verformung des Dichtungslippenabschnitts 8.1, da die Dicke dieses Abschnitts im Normalzustand exakt vorgegeben und die Breite des Einbauspaltes 5.3 durch einen metallischen Anschlag exakt eingehalten werden kann.

Durch das Einspannen des Dichtungslippenabschnittes 8.1 ist ein sicherer Halt der Lippendichtung 8 auch ohne Verwendung von Klebstoffen gewährleistet, wodurch die Montage wesentlich erleichtert wird.

### Bezugszeichenliste

- 1: Sichtfenster
- 2: Trennwand
- 3: Rein- oder Sterilraum
- 4: Umgebung
- 5: Fenster- oder Einbaurahmen
- 6: Einbaurahmenöffnung
- 7: Scheibe
- 7.1: Scheibeninnenseite
- 7.2: Scheibenaußenseite
- 8: Lippendichtung
- 8.1, 8.2: Lippendichtungsabschnitt
- 8.1.1: Rand
- 8.1.2: Abschrägung
- 8.2.3: Dichtungsrand
- 8.2.2: Fläche
- 8.3: Wulst
- 9: Riegel
- 10: Spalt
- 11: Bolzen
- 11.1: Innengewinde
- 11.2: Bolzenkopf
- 12: Spannschraube
- 13: Druckfeder
- 14: Stützelement

## Patentansprüche

1. Sichtfenster einer einen Rein- oder Sterilraum (3) bildenden Einhausung (2) für eine sterile und/oder aseptische Behandlung von Packmitteln, mit wenigstens einem Einbaurahmen (5) und mit einer eine Einbaurahmenöffnung (6) verschließenden Scheibe (7), mit wenigstens einer den Übergang zwischen der Scheibe (7) und dem Einbaurahmen (5) abdichtenden, umlaufenden Lippendichtung (8) sowie mit Mitteln (9, 14) zum Halten der Scheibe (7) am Einbaurahmen (5), wobei die wenigstens eine Lippendichtung (8) einstückig und als Vollprofil aus einem gummi- oder dauerelastischen Material mit einem zumindest teilweise sichelartigen Profilquerschnitt mit dem ersten, leistenartigen Lippendichtungsabschnitt (8.1) und mit einem zweiten Lippendichtungsabschnitt (8.2) hergestellt ist, wobei der Profilquerschnitt des zweiten Lippendichtungsabschnitts (8.2) zumindest in einem an den ersten Lippendichtungsabschnitt (8.1) anschließenden ersten Teilabschnitt (8.2.1) um wenigstens eine in Längsrichtung der Lippendichtung orientierte Achse gekrümmt ist, wobei der zweite Lippendichtungsabschnitt (8.2) einen dem ersten, Lippendichtungsabschnitt (8.1) entfernten Dichtungslippenrand (8.2.3) bildet, wobei die Dicke des zweiten Lippendichtungsabschnitts (8.2) zu dem Dichtungslippenrand (8.2.3) hin abnimmt, wobei der Profilquerschnitt am ersten Teilabschnitt (8.2.1) an einer erste Seite um wenigstens eine in Längsrichtung der Lippendichtung orientierte Achse konvex und an einer zweiten Seite konkav gekrümmt ist,
wobei der erste Lippendichtungsabschnitt (8.1) als Vollprofil leistenartig ausgebildet ist,
**dadurch gekennzeichnet, dass**
der Profilquerschnitt des zweiten Lippendichtungsabschnitts (8.2) in einem zweiten Teilabschnitt (8.2 2), der an den ersten Teilabschnitt (8.2.1) anschließt und den Dichtungslippenrand (8.2.3) aufweist, an der ersten Seite um wenigstens eine in Längsrichtung der Lippendichtung (8) orientierte Achse konkav und an der zweiten Seite konvex gekrümmt ist wobei die Lippendichtung (8) an ihrem zweiten Lippendichtungsabschnitt (8.2) elastisch verformt mit dem Dichtungslippenrand (8.2.3) gegen eine dem Rein- oder Sterilraum (3) zugeordnete Scheibeninnenseite (7.1) der Scheibe (7) anliegt, wobei die Lippendichtung (8) mit ihrem ersten Lippendichtungsabschnitt (8.1) elastisch verformt in einem Einbauspalt (5.3) zwischen zwei Einspannflächen des Einbaurahmens (5) umlaufend eingespannt ist und mit ihrem zweiten Lippendichtungsabschnitt (8.2) aus dem Einbauspalt (5.3) über einen Innenrand der Einbaurahmenöffnung (6) vorsteht.

2. Sichtfenster nach Anspruch 1, **dadurch gekennzeichnet, dass** der Einbaurahmen (5) aus wenigstens zwei Rahmenteilen (5.1, 5.2) besteht, die zwischen sich den Einbauspalt (5.3) bilden.

3. Sichtfenster nach Anspruch 1 oder 2, **dadurch gekennzeichnet, dass** der Einbaurahmen (5) dazu eingerichtet ist, eine dem Rein- oder Sterilraum (3) zugewandte Innenseite und eine einer Umgebung (4) zugewandte Außenseite auszubilden, und die Scheibe (7) mit ihrer Scheibeninnenseite (7.1) der Außenseite des Einbaurahmens (5) zugewandt, aber von dieser Ebene beabstandet ist.

4. Sichtfenster nach einem der vorhergehenden Ansprüche, **dadurch gekennzeichnet, dass** der erste Lippendichtungsabschnitt (8.1) durch das Einspannen zwischen den Rahmenteilen (5.1, 5.2) auf 70% - 80% seiner ursprünglichen Dicke elastisch verformt ist.

5. Sichtfenster nach einem der vorhergehenden Ansprüche, **dadurch gekennzeichnet, dass** der Dichtungslippenrand (8.1.2) gegen die Scheibeninnenseite(7.1) im Abstand vom Umfangsrand (7.3) der Scheibe (7) und im Abstand von dem Innenrand der Einbaurahmenöffnung (6) anliegt.

6. Sichtfenster nach einem der vorhergehenden Ansprüche, **dadurch gekennzeichnet, dass** die dem Rein- oder Sterilraum zugewandte Fläche (8.2.2) der Lippendichtung (8) im Bereich des gegen die Scheibeninnenseite (7.1) anliegenden Dichtungsrandes (8.2.3) mit einer Ebene der Scheibeninnenseite (7.1.1) einen Winkel größer als 90° einschließt.

7. Sichtfenster nach einem der vorhergehenden Ansprüche, **gekennzeichnet durch** eine Vielzahl von jeweils mit einem Muttergewindestück (11) zusammenwirkenden Spannschrauben (12) zum Verspannen des ersten Lippendichtungsabschnitts (8.1) zwischen den Rahmenteilen (5.1, 5.2).

8. Sichtfenster nach einem der vorhergehenden Ansprüche, **dadurch gekennzeichnet, dass** die Mittel zum Halten der Scheiben (7) von einer Vielzahl von Riegeln (9) gebildet sind, die gegen die Scheibenaußenseite (7.2) anliegen, und dass am Einbaurahmen (5) vorzugsweise zusätzlich Stützelemente (14) vorgesehen sind, gegen die die Scheibe (7) mit ihrem Umfangsrand (7.3) anliegt.

9. Einhausung zur Erzielung eines von einer Umgebung (4) getrennten Rein- oder Sterilraumes (3) zur sterilen und/oder aseptischen Behandlung von Packmitteln, beispielsweise zum sterilen und/oder aseptischen Füllen und Verschließen von Packmitteln, mit wenigstens einem Sichtfenster (1) in einer den Rein- oder Sterilraum (3) von der Umgebung (4) trennenden Wand (2), **dadurch gekennzeichnet, dass** das Sichtfenster nach einem der Ansprüche 1 - 8 ausgebildet ist.

## Claims

1. Viewing window of an enclosure (2) forming a clean or sterile space (3) for a sterile and/or aseptic treatment of packaging means, with at least one mounting frame (5) and with a pane (7) closing a mounting frame opening (6), with at least one circumferential lip seal (8) sealing the transition between the pane (7) and the mounting frame (5), as well as with means (9, 14) for holding the pane (7) at the mounting frame (5), wherein the at least one lip seal (8) is produced as one piece and as a full profile, from a rubber material or permanently elastic material, with an at least partially sickle-shaped profile cross-section, with the first strip-shaped lip seal section (8.1) and with a second lip seal section (8.2), wherein the profile cross-section of the second lip seal section (8.2) is cambered at least in one first part section (8.2.1), connecting to the first lip seal section (8.1), about at least one axis oriented in the longitudinal direction of the lip seal, wherein the second lip seal section (8.2) forms a first lip seal edge (8.2.3) remote from the first lip seal section (8.1), wherein the thickness of the second lip seal section (8.2) decreases towards the lip seal edge (8.2.3), wherein the profile cross-section at the first part section (8.2.1) is cambered on a first side as convex at least one axis oriented in the longitudinal direction of the lip seal, and as concave on a second side, wherein the first lip seal section (8.1) is configured as full-profile in strip form, **characterised in that**
the profile cross-section of the second lip seal section (8.2) in a second part section (8.2.2), which connects to the first part section (8.2.1) and comprises the lip seal edge (8.2.3), is cambered on the first side as concave about at least one axis oriented in the longitudinal direction of the lip seal (8), and as convex on the second side, wherein the lip seal (8) is elastically deformed at its second lip seal section (8.2) with the seal lip edge (8.2.3) in contact against a pane inner side (7.1) of the pane (7), assigned to the clean or sterile space (3), wherein the lip seal (8), with its first lip seal section (8.1) elastically deformed, is circumferentially tensioned in a mounting gap (5.3) between two tensioning surfaces of the mounting frame (5), and with its second lip seal section (8.2) projects out of the mounting gap (5.3) over an inner edge of the mounting frame opening (6).

2. Viewing window according to claim 1, **characterised in that** the mounting frame (5) consists of at least two frame parts (5.1, 5.2), which form between them the mounting gap (5.3).

3. Viewing window according to claim 1 or 2, **characterised in that** the mounting frame (5) is configured such as to form an inner side facing towards the clean or sterile space (3), and an outer side facing towards the surrounding environment (4), and the pane (7) faces with its pane inner side (7.1) facing towards the outer side of the mounting frame (5), but is spaced at a distance from this plane.

4. Viewing window according to any one of the preceding claims, **characterised in that** the first lip seal section (8.1) is elastically deformed by the tensioning between the frame parts (5.1, 5.2) to 70% - 80% of its original thickness.

5. Viewing window according to any one of the preceding claims, **characterised in that** the sealing lip edge (8.1.2) is in contact against the pane inner side (7.1), at a distance interval from the circumferential edge (7.3) of the pane (7) and at a distance interval from the inner edge of the mounting frame opening (6).

6. Viewing window according to any one of the preceding claims, **characterised in that** the surface (8.2.2) of the lip seal (8) facing towards the clean or sterile space, in the region of the seal edge (8.2.3) in contact against the inner side of the pane (7.1), encloses with a plane of the pane inner side (7.1.1) an angle greater than 90°.

7. Viewing window according to any one of the preceding claims, **characterised by** a plurality of tightening screws (12), interacting with a threaded nut element (bolt) (11), for tensioning the first lip seal section (8.1) between the frame parts (5.1, 5.2).

8. Viewing window according to any one of the preceding claims, **characterised in that** the means for holding the panes (7) are formed from a plurality of latches (9), which are in contact against the pane outer side (7.2), and that preferably additional support elements (14) are provided at the mounting frame (5), against which the pane (7) is in contact with its circumferential edge (7.3).

9. Enclosure for obtaining a clean or sterile space (3), separated from an outside environment (4), for the sterile and/or aseptic treatment of packaging means, for example for the sterile and/or aseptic filling and closing of packaging means, with at least one viewing window (1) in a wall (2), separating the clean or sterile space (3) from the surrounding environment (4), **characterised in that** the viewing window is configured in accordance with any one of claims 1-8.

## Revendications

1. Fenêtre d'inspection d'une enceinte (2) formant une salle blanche ou stérile (3) pour traiter de manière stérile et/ou aseptisée des moyens d'emballage, avec au moins un châssis de montage (5) et une vitre (7) fermant une ouverture de châssis de montage (6), avec au moins un joint à lèvre (8) périphérique étanchéifiant le passage entre la vitre (7) et le châssis de montage (5) ainsi qu'avec des moyens (9, 14) servant à maintenir la vitre (7) au niveau du châssis de montage (5), dans laquelle l'au moins un joint à lèvre (8) est fabriqué d'un seul tenant et en tant que profil plein composé d'un matériau ayant une élasticité de type caoutchouc ou permanente avec une section transversale de profil au moins en partie de type demi-lune avec la première section de joint à lèvre (8.1) de type baguette et avec une deuxième section de joint à lèvre (8.2), dans lequel la section transversale de profil de la deuxième section de joint à lèvre (8.2) est incurvée au moins dans une première section partielle (8.2.1) se raccordant à la première section de joint à lèvre (8.1) autour d'au moins un axe orienté dans le sens longitudinal du joint à lèvre, dans lequel la deuxième section de joint à lèvre (8.2) forme un bord de lèvre de joint (8.2.3) éloigné de la première section de joint à lèvre (8.1), dans lequel l'épaisseur de la deuxième section de joint à lèvre (8.2) diminue en direction du bord de lèvre de joint (8.2.3), dans lequel la section transversale de profil est incurvée au niveau de la première section partielle (8.2.1) de manière convexe au niveau d'un premier côté autour d'au moins un axe orienté dans le sens longitudinal du joint à lèvre et de manière concave au niveau d'un deuxième côté,
dans lequel la première section de joint à lèvre (8.1) est réalisée en tant que profil plein de type baguette,
**caractérisée en ce que**
la section transversale de profil de la deuxième section de joint à lèvre (8.2) est incurvée dans une deuxième section partielle (8.2.2), qui se raccorde à la première section partielle (8.2.1) et présente le bord de lèvre de joint (8.2.3), de manière concave au niveau du premier côté autour d'au moins un axe orienté dans le sens longitudinal du joint à lèvre (8), et de manière convexe au niveau du deuxième côté, dans lequel le joint à lèvre (8) repose, de manière déformée élastiquement au niveau de sa deuxième section de joint à lèvre (8.2), avec le bord de lèvre de joint (8.2.3) contre un côté intérieur de vitre (7.1) de la vitre (7) associé à la salle blanche ou stérile (3), dans lequel le joint à lèvre (8) est enserré en périphérie de manière déformée élastiquement avec sa première section de joint à lèvre (8.1) dans une fente de montage (5.3) entre deux surfaces d'enserrage du châssis de montage (5) et dépasse par sa deuxième section de joint à lèvre (8.2) hors de la fente de montage (5.3) au-delà d'un bord intérieur de l'ouverture de châssis de montage (6).

2. Fenêtre d'inspection selon la revendication 1, **caractérisée en ce que** le châssis de montage (5) est constitué d'au moins deux parties de châssis (5.1, 5.2), qui forment entre elles la fente de montage (5.3).

3. Fenêtre d'inspection selon la revendication 1 ou 2, **caractérisée en ce que** le châssis de montage (5) est conçu pour réaliser un côté intérieur tourné vers la salle blanche ou stérile (3) et un côté extérieur tourné vers un environnement (4), et la vitre (7) est tournée vers le côté extérieur du châssis de montage (5) par son côté intérieur de vitre (7.1), mais est toutefois espacée dudit plan.

4. Fenêtre d'inspection selon l'une quelconque des revendications précédentes, **caractérisée en ce que** la première section de joint à lèvre (8.1) est déformée élastiquement par l'enserrage entre les parties de châssis (5.1, 5.2) sur 70 % à 80 % de son épaisseur d'origine.

5. Fenêtre d'inspection selon l'une quelconque des revendications précédentes, **caractérisée en ce que** le bord de lèvre de joint (8.1.2) repose contre le côté intérieur de vitre (7.1) à distance du bord périphérique (7.3) de la vitre (7) et à distance du bord intérieur de l'ouverture de châssis de montage (6).

6. Fenêtre d'inspection selon l'une quelconque des revendications précédentes, **caractérisée en ce que** la surface (8.2.2) du joint à lèvre (8) tournée vers la salle blanche ou stérile forme, dans la zone du bord de joint (8.2.3) reposant contre le côté intérieur de vitre (7.1), un angle supérieur à 90° avec un plan du côté intérieur de vitre (7.1.1).

7. Fenêtre d'inspection selon l'une quelconque des revendications précédentes, **caractérisée par** une pluralité de vis de serrage (12) coopérant respectivement avec une pièce à filetage femelle (11) servant à assembler par serrage la première section de joint à lèvre (8.1) entre les parties de châssis (5.1, 5.2).

8. Fenêtre d'inspection selon l'une quelconque des revendications précédentes, **caractérisée en ce que** les moyens servant à maintenir les vitres (7) sont formés par une pluralité de verrous (9), qui reposent contre le côté extérieur de vitre (7.2), et que sont prévus au niveau du châssis de montage (5) de préférence en supplément des éléments d'appui (14), contre lesquels la vitre (7) repose par son bord périphérique (7.3).

9. Enceinte servant à obtenir une salle blanche ou stérile (3) séparée d'un environnement (4) servant à traiter de manière stérile et/ou aseptisée des moyens d'emballage, par exemple servant à remplir et à fermer de manière stérile et/ou aseptisée des moyens d'emballage, avec au moins une fenêtre d'inspection (1) dans une paroi (2) séparant la salle blanche ou stérile (3) de l'environnement (4), **caractérisée en ce que** la fenêtre d'inspection est réalisée selon l'une quelconque des revendications 1 à 8.
